**Europäisches Patentamt**

(19) **European Patent Office**

**Office européen des brevets**

(11) Veröffentlichungsnummer: **0 123 157**
**B1**

(12) **EUROPÄISCHE PATENTSCHRIFT**

(45) Veröffentlichungstag der Patentschrift:
24.06.87

(51) Int. Cl.⁴: **A 61 K 31/54** // (A61K31/54, 31:505, 31:47)

(21) Anmeldenummer: 84103280.8

(22) Anmeldetag: 26.03.84

(54) **Arzneimittel und/oder Futtermittelkonzentrate mit antibakterieller Wirkung.**

(30) Priorität: 25.03.83 HU 101183

(43) Veröffentlichungstag der Anmeldung:
31.10.84 Patentblatt 84/44

(45) Bekanntmachung des Hinweises auf die Patenterteilung:
24.06.87 Patentblatt 87/26

(84) Benannte Vertragsstaaten:
AT BE CH DE FR GB IT LI NL SE

(56) Entgegenhaltungen:
EXPERIENTIA, Band 31, Fasc. 4, 15. April 1975, Seiten 444-445, Basel, CH; J. MOLNAR et al.: "The antibacterial action and R-factor-inhibiting activity by chlorpromazine"
CHEMICAL ABSTRACTS, Band 85, Nr. 3, 19. Juli 1976, Seite 115, Nr. 14610r, Columbus, Ohio, USA; J. MOLNAR et al.: "Antibacterial effect of some phenothiazine compounds and R-factor elimination by chlor promazine"
CHEMICAL ABSTRACTS, Band 88, Nr. 11, 13. März 1978, Seite 97, Nr. 69669j, Columbus, Ohio, USA; S. YAMABE: "Synergistic effects of chlorpromazine and perphenazine on several chemotherapeutic agents. I. General profile of the effects measured by the filter paper strip-agar diffusion method with Escherichia coli and Pseudomonas aeruginosa"

(73) Patentinhaber: EGIS Gyogyszergyár, Kereszturi ut 30-38, Budapest X (HU)

(72) Erfinder: Magyar, Károly, Dr., Attila u. 131, Budapest I. (HU)
Erfinder: Kelemen, Jozsef, Dr., Tulipán u. 10, Budapest II (HU)
Erfinder: Benko, Pál, Dr., Tartsay u. 7, Budapest XII (HU)
Erfinder: Simon, Ferenc, Dr., Szakasits u. 64/b, Budapest XI (HU)
Erfinder: Varga, János, Dr., Bokányi u. 88, Budapest XVIII (HU)
Erfinder: Romváry, Attila, Dr., Bimbo u. 141/a, Budapest II (HU)
Erfinder: Egri, János, Dereglye u. 2, Budapest III (HU)
Erfinder: Bozsing, Dániel, Vezér u. 116, Budapest XIV (HU)

(74) Vertreter: Beszédes, Stephan G. Dr., Münchener Strasse 80a Postfach 1168, D-8060 Dachau (DE)

(56) Entgegenhaltungen: (Fortsetzung)
UNLISTED DRUGS, Band 30, Dezember 1978, Chatham, New Jersey, USA; Vetoprim (Kartei)
UNLISTED DRUGS, Band 29, Nr. 3, März 1977, Chatham, New Jersey, USA; Seite 38 L "Ufa 902-Duo"
UNLISTED DRUGS, Band 22, Nr. 4, April 1970, Chatham, New Jersey, USA; Seite 59 L "Trivetrin"
UNLISTED DRUGS, Band 25, Nr. 8, August 1973, Chatham, New Jersey, USA; Seite 131 R "Sumetrolim"

## Beschreibung

Die Erfindung betrifft neue Arzneimittel mit antibakterieller Wirkung, die besonders gut in der Veterinärmedizin anwendbar sind, und Futtermittelkonzentrate mit antibakterieller Wirkung.

Die bakterielle Infektion von neugeborenen Tieren bereitet in der Tierzucht sehr grosse wirtschaftliche Probleme. Die Colitoxikämie und Enteritis bei Kälbern und die Enterotoxikämie und sogenannte Ödemkrankheit bei Absatzferkeln sind die am häufigsten vorkommenden Infektionen dieser Art.

Zur Behandlung der Colitoxikämie von Kälbern und Absatzferkeln werden heutzutage Arzneimittelpräparate, die im allgemeinen Sulfonamide (allein oder in Kombination mit 2,4-Diamino -5- [3', 4', 5'-tri-(methoxy)-benzyl]-pyrimidin {Trimethoprim}, Antibiotica beziehungsweise Nitrofuran- oder Nitroimidazolderivate als Wirkstoffe enthalten, verwendet. Allmählich wurden aber die Bakterien gegen diese Verbindungen resistent, weswegen die bekannten Arzneimittelpräparate nur mit sehr mässigem Erfolg in der Therapie von Colitoxikämie verwendet werden können. Auch die mit Vaccinen durchgeführten Behandlungen geben keine günstigeren Ergebnisse.

In der Therapie der Erkrankungen der Verdauungsorgane sollen die folgenden Forderungen erfüllt werden:

A) Die Infektionserreger sind zu vernichten und

B) die physiologischen und biochemischen Vorgänge, die durch die pathogenen Keime oder deren Produkte (Toxine) in abnormaler Richtung beeinflusst wurden, sollen normalisiert werden.

Der Erfindung liegt die Aufgabe zugrunde, Arzneimittel und/oder Futtermittelkonzentrate mit antibakterieller Wirkung, mit denen die genannten Erkrankungen erfolgreicher behandelt werden können, zu schaffen.

Das Obige wurde überraschenderweise durch die Erfindung erreicht.

Gegenstand der Erfindung sind Arzneimittel und/oder Futtermittelkonzentrate mit antibakterieller Wirkung, mit einem Gehalt an

a) 2,4-Diamino-5-[3',4',5'-tri-(methoxy)-benzyl]-pyrimidin {Trimethoprim}, gegebenenfalls zusammen mit 1 oder mehr in Arzneimitteln und/oder Futtermittelkonzentraten üblichen Konfektionierungsmittel(n) und/oder 1 oder mehr Salicylsäurederivat(en), welche dadurch gekennzeichnet sind, dass sie als weitere Wirkstoffe

b) 2-Chlor-10-[3-(dimethylamino)-propyl]-phenothiazin {Chlorpromazin} und/oder 1 oder mehr physiologisch verträgliche[s] Säureadditionssalze(e) desselben und

c) 2-(Methyl)-5,7-di-(chlor)-8-(hydroxy)-chinolin {Chlorchinaldol} enthalten.

In «Experientia», Bd. 31, Fasc. 4, 15. April 1975 wird auf den Seiten 444 bis 445 die bakterienwachstumshemmende Wirkung von Chlorpromazin in vergleichender Gegenüberstellung zu der entsprechenden Wirkung von Levomepromazin und Promethazin beschrieben. Ein Hinweis auf irgendeine synergistische Wirkung ist dieser Arbeit, die auch in «Chemical Abstracts», Bd. 85, Nr. 3, 19. Juli 1976 auf Seite 115 unter der Nummer 14610r, referiert wird, nicht zu entnehmen.

In «Chemical Abstracts», Bd. 88, Nr. 11, 13. März 1978, wird auf Seite 97 unter der Nr. 69669j über die synergistischen Wirkungen von Chlorpromazin und Perphenazin auf chemotherapeutische Mittel, wie β-Lactam-Antibiotika (Ampicillin), berichtet. Ein Hinweis auf die erfindungsgemässe Feststellung, dass eine Kombination aus Trimethoprim und Chlorpromazin und/oder Chlorchinaldol eine ausgeprägte synergistische Wirkung besitzt, ist dieser Veröffentlichung nicht zu entnehmen.

Die Literaturstelle «Unlisted Drugs», Bd. 30, Dezember 1978, erwähnt ein auf dem Veterinärgebiet eingesetztes Pulver aus Trimethoprim, Sulfamethazin, Sulfathiazol, Furazolidon, Vitamin-A-palmitat und noch weiteren Bestandteilen und gibt ebenfalls keinen Hinweis auf die erfindungsgemässe synergistische Kombination. Das gleiche gilt für die in der Literaturstelle «Unlisted Drugs», Bd. 29, Nr. 3, März 1977, beschriebene Kombination aus Trimethoprim, Sulfamethazin und Sulfathiazol, für die in «Unlisted Drugs», Bd. 22, Nr. 4, April 1970, beschriebene Kombination aus Sulfadoxin und Trimethoprim sowie die in «Unlisted Drugs», Bd. 25, Nr. 8, August 1973, offenbarte Kombination aus Sulfamethoxazol und Trimethoprim.

Vorzugsweise enthalten die erfindungsgemässen Arzneimittel als physiologisch verträgliches Säureadditionssalz von 2-Chlor-10-[3-(dimethylamino)-propyl]-phenthiazin {Chlorpromazin} <b> 2-Chlor-10-[3-(dimethylamino)-propyl]-phenothiazin-hydrochlorid {Chlorpromazinhydrochlorid}.

Zweckmässig ist in den erfindungsgemässen Arzneimitteln und/oder Futtermittelkonzentraten das Gewichtsverhältnis von a):b):c) 6:1:6 bis 110:1:100, insbesondere 6: 1:6 bis 50:1:100, ganz besonders 20:1:20 bis 40:1:40 beziehungsweise 90:1:20 bis 110:1:40. Aber auch erfindungsgemässe Arzneimittel und/oder Futtermittelkonzentrate beispielsweise mit Gewichtsverhältnissen von a):b):c) von 1:10:50 bis 1:10:1 sind gut wirksam. Die Gewichtsverhältnisbereiche sind so zu verstehen, dass die Gewichtsverhältniswerte von a) und c) unabhängig von dem des jeweiligen anderen von ihrem jeweiligen unteren bis zu ihrem jeweiligen oberen variieren können.

Es wurde nämlich überraschenderweise festgestellt, dass die gleichzeitige Verwendung von 2,4-Diamino-5-[3', 4', 5'-tri-(methoxy)-benzyl]-pyrimidin {Trimethoprim} <a)>, von 2-Chlor-10-[3-(dimethylamino)-propyl]-phenothiazin {Chlorpromazin} und/oder 1 oder mehr physiologisch verträgliche[s] Säureadditionssalz(e), insbesondere des Hydrochlorides, desselben <b)> und 2-(Methyl)-5,7-di-(chlor) -8- (hydroxy)-chinolin {Chlorchinaldol} <c)> eine synergistische Potenzierung der antibakteriellen Wirkung sichert. Die Mindesthemmkonzentration (MIC-Werte) der Kombination der 3 obigen Wirkstoffe und der einzelnen obigen Wirkstoffe wurden in vitro gegen die Bakterienstämme Escherichia coli 494, Bordatella bronchiseptica, Staphylococcus aureus und Listeria monocytogenes in einer Phenolrot und Glucose enthaltenden Brühe [Hersteller: Firma Difca] bestimmt. In der folgenden Tabelle sind die Werte der Mindesthemmkonzentration (MIC-Werte] in mg/l, die den Bakterienzuwachs 24 Stunden lang hemmen, zusammengestellt.

Tabelle

| Untersuchtes Material | | Werte der Mindesthemmkonzentration [MIC-Werte] in mg/l in Bezug auf die Bakterienstämme | | | |
|---|---|---|---|---|---|
| Bezeichnung | Menge in mg/l | Gegen 2,2-Dichlor-N-[(αR, βR)-β-hydroxy-α-hydroxymethyl-4-nitrophenäthyl]-acetamid {Chloramphenicol} resistenter Stamm Escherichia coli 494 | Bordatella bronchiseptica | Staphylococcus aureus | Listeria monocytogenes |
| Erfindungsgemässes Arzneimittelpräparat, bestehend aus | | | | | |
| 2,4-Diamino-5-[3',4',5'-tri-(methoxy)-benzyl]-pyrimidin {Trimethoprim} <a)> | 5 | 5 | 5 | 5 | 5 |
| 2-Chlor-10-[3-(dimethylamino)-propyl]-phenothiazin {Chlorpromazin} <b)> | 0,5 | 0,5 | 0,5 | 0,5 | 0,5 |
| 2-(Methyl)-5,7-di-(chlor)-8-(hydroxy)-chinolin {Chlorchinaldol} <c)> | 25*) bzw. 0,5**) | 25 | 0,5 | 0,5 | 0,5 |
| 2,4-Diamino-5-[3',4',5'-tri-(methoxy)-benzyl]-pyrimidin {Trimethoprim} <Vergleichssubstanz A> | 25△ 50△△ 100△△△ | 25 | 50 | 100 | 50 |
| 2-Chlor-10-[3-(dimethylamino)-propyl]-phenothiazin {Chlorpromazin} <Vergleichssubstanz B> | 50° bzw. 200°° bzw. 25°°° | 50 | 200 | 25 | 25 |
| 2-(Methyl)-5,7-di-(chlor)-8-(hydroxy)-chinolin {Chlorchinaldol} <Vergleichssubstanz C> | 200▽ 5▽▽ | 200 | 5 | 5 | 5 |

△    gegen 2,2-Dichlor-N-[(αR, βR)-β-hydroxy-α-hydroxymethyl-4-nitrophenäthyl]-acetamid {Chloramphenicol} resistenter Stamm Escherichia coli 494

△△    gegen Bordatella bronchiseptica und Listeria monocytogenes

△△△ gegen Staphylococcus aureus

°    gegen 2,2-Dichlor-N-[(αR, βR)-β-hydroxy-α-hydroxymethyl-4-nitrophenäthyl]-acetamid {Chloramphenicol} resistenter Stamm Escherichia coli 494

°°    gegen Bordatella bronchiseptica

°°°    gegen Staphylococcus aureus und Listeria monocytogenes

▽    gegen 2,2-Dichlor-N-[(αR, βR)-β-hydroxy-α-hydroxymethyl-4-nitrophenäthyl]-acetamid {Chloramphenicol} resistenter Stamm Escherichia coli 494

▽▽    gegen Bordatella bronchiseptica, Staphylococcus aureus und Listeria monocytogenes

*    gegen 2,2-Dichlor-N-[(αR, βR)-β-hydroxy-α-hydroxymethyl-4-nitrophenäthyl]-acetamid {Chloramphenicol} resistenter Stamm Escherichia coli 494

**    gegen Bordatella bronchiseptica, Staphylococcus aureus und Listeria monocytogenes

Aus der obigen Tabelle geht hervor, dass die einzelnen Wirkstoffe das Wachstum der geprüften Bakterienstämme nur in verhältnismässig hohen Dosen hemmen. Im Falle des synergistischen erfindungsgemässen Arzneimittels wird dagegen die gleiche Wirkung durch $\frac{1}{20}$ bis $\frac{1}{5}$ der Einzelkonzentration von 2,4-Diamino -5- [3', 4', 5'-tri-(methoxy)-benzyl]-pyrimidin [Trimethoprim} <a)>, $\frac{1}{50}$ bis $\frac{1}{400}$ der Einzelkonzentration von 2-Chlor -10- [3-(dimethylamino)-propyl]-phenothiazin [Chlorpromazin} <b)> und $\frac{1}{10}$ bis $\frac{1}{8}$ der Einzelkonzentration von 2-(Methyl)-5,7-di-(chlor) -8- (hydroxy)-chinolin [Chlorchinaldol} <c)> erreicht. Auch das Wachstum des gegen 2,2-Dichlor-N-[(αR, βR)-β-hydroxy-α-hydroxymethyl -4- nitrophenäthyl]-acetamid [Chloramphenicol} resistenten Stammes Escherichia coli 494 wird durch das synergistische erfindungsgemässe Arzneimittel gehemmt.

Als Konfektionierungsmittel können die erfindungsgemässen Arzneimittel und/oder Futtermittelkonzentrate in Arzneimittelpräparaten und/oder Futtermittelkonzentraten übliche Träger enthalten.

Die erfindungsgemässen Arzneimittel können in Form von Arzneimittelpräparaten, die im allgemeinen in der Therapie und besonders in der Veterinärpraxis üblich sind, zum Beispiel als Tabletten, überzogene Tabletten, Pulvergemische, Injektionslösungen, Pillen, Suspensionen beziehungsweise Emulsionen vorliegen. Vorteilhafte Arzneimittelpräparate sind Pulvergemische und Suspensionen.

Die erfindungsgemässen Arzneimittelpräparatpulvergemische können neben den Wirkstoffen 1 oder mehr Träger, vorteilhaft Glucose, Nicotinamid, Hexamethylentetramin, Polyvinylpyrrolidon, Silikate, zum Beispiel Aluminiumsilikat und/oder Aluminiummagnesiumsilikat und/oder Siliciumdioxyd und/oder 1 oder mehr Salicylsäurederivat(e), zum Beispiel Acetylsalicylsäure und/oder Wismutsubsalicylat, enthalten.

Vorteilhafte Träger in den erfindungsgemässen Arzneimittelpräparatsuspensionen sind Wasser und/oder organische Lösungsmittel, Emulgiermittel, Dispergiermittel, Suspendiermittel, Netzmittel, Antioxydationsmittel, Stabilisatoren und/oder Süssungsmittel.

Neben den Wirkstoffen können die erfindungsgemässen Futtermittelkonzentrate im allgemeinen 1 oder mehr feste[n] Träger, zum Beispiel Weizenfuttermehl, Weizenkleie, entölte Reiskleie, Maismehl, Sojamehl, Kaolin, Zeolithe und/oder Calciumcarbamat, und/oder flüssige[n] Träger, zum Beispiel Wasser, physiologisch anwendbare Salzlösung(en) und/oder organische[s] Lösungsmittel, wie Polyäthylenglykole, und/oder ferner Emulgiermittel, Dispergiermittel, Suspendiermittel, Netzmittel, Antioxydationsmittel, Spurenelement(e), Vitamin(e) und/oder anorganische[s] Salz(e), enthalten.

In den erfindungsgemässen Arzneimittelpräparaten und/oder Futtermittelkonzentraten beträgt die Gesamtmenge der Wirkstoffe meistens 1 bis 90 Gew.-%, vorteilhaft 5 bis 20 Gew.-%. Die auf 1 kg Körpergewicht bezogene tägliche Dosis ist zweckmässig 5 bis 150 mg Gemisch der 3 Wirkstoffe.

Die erfindungsgemässen Arzneimittelpräparate und/oder Futtermittelkonzentrate können in an sich bekannter Weise durch Vermischen der Wirkstoffe mit 1 oder mehr Konfektionierungsmittel(n) bereitet werden. Besonders wenn Pulvergemische oder Futtermittelkonzentrate bereitet werden, werden die erhaltenen Gemische, falls es erwünscht ist, gemahlen.

Die erfindungsgemässen Arzneimittelpräparatsuspensionen können unmittelbar vor ihrer Verabreichung bereitet werden, indem die Arzneimittelpräparatpulvergemische in entsprechenden Flüssigkeiten suspendiert werden.

Gegenstände der Erfindung sind auch Erzeugnisse, enthaltend 2,4-Diamino -5- [3', 4', 5'-tri-(methoxy)-benzyl]-pyrimidin [Trimethoprim} <a)> und gegebenenfalls 1 oder mehr in Arzneimitteln und/oder Futtermittelkonzentraten übliche Konfektionierungsmittel, welche dadurch gekennzeichnet sind, dass sie ausser <a)> als weitere Wirkstoffe 2-Chlor -10- [3-(dimethylamino)-propyl]-phenothiazin [Chlorpromazin} <b)> und/oder 1 oder mehr physiologisch verträgliche[s] Säureadditionssalz(e) desselben <b)> und 2-(Methyl)-5,7-di-(chlor) -8- (hydroxy)-chinolin [Chlorchinaldol} <c)> als Kombinationspräparat zur gleichzeitigen, getrennten oder zeitlich abgestuften Anwendung in der antibakteriellen Therapie enthalten. Für die erfindungsgemässen Erzeugnisse gelten dieselben Bevorzugungen wie für die erfindungsgemässen Arzneimittel und Futtermittelkonzentrate.

Die Wirkstoffe der erfindungsgemässen Arzneimittel und/oder Futtermittelkonzentrate sind bekannte handelsübliche Verbindungen.

Die erfindungsgemässen Arzneimittel werden den Tieren im allgemeinen peroral verabreicht.

Die erfindungsgemässen Futtermittelkonzentrate können zum Bereiten von gebrauchsfertigen Tierfuttermitteln mit entsprechenden Futtermittelbestandteilen verdünnt und/oder mit entsprechenden Tierfuttermitteln vermischt werden. Sie können auch mit Trinkwasser vermischt werden. Die im Tierfuttermittel beziehungsweise Trinkwasser vorhandenen Wirkstoffmengen sollen die für die erfolgreiche Behandlung notwendige tägliche Dosis sichern.

Die bakteriellen Infektionen, besonders die Colitoxikämie und Enteritis bei Kälbern und die Enterotoxikämie bei Absatzferkeln, können mit den erfindungsgemässen Arzneimitteln und/oder Futtermittelkonzentraten wirksam behandelt werden, und zwar auch in den Fällen, in welchen die krankheitserregenden Bakterienstämme gegen Antibiotica resistent sind.

Die Erfindung wird an Hand der folgenden Beispiele näher erläutert.

**Beispiel 1**

Es wurden 0,5 g 15 mg 2,4-Diamino -5- [3', 4', 5'-tri-(methoxy)-benzyl]-pyrimidin {Trimethoprim} <a)>, 2-Chlor -10- [3-(dimethylamino)-propyl]-phenothiazin {Chlorpromazinhydrochlorid} <b)>, 15 g 2-(Methyl)-5,7-di-(chlor) -8- (hydroxy)-chinolin {Chlorchinaldol} <c)>, 60 g Acetylsalicylsäure, 50 g Glucose, 20 g Nicotinamid, 35,5 g Stärke und 4 g Siliciumdioxyd miteinander vermischt und in einer Hammermühle gemahlen.

Kälber mit einem Körpergewicht von 50 kg wurden mit je 5 g Portionen des erhaltenen Pulvergemisches 2mal täglich behandelt. Jede 5 g Portion des Pulvergemisches wurde vor der Verabreichung in 500 ml Kamillentee suspendiert und die gut aufgerührte Suspension wurde den Tieren peroral verabreicht. Die Behandlung dauerte 3 Tage.

Ferkel mit einem Körpergewicht von 5 kg wurden mit einer 1 g Portion des Pulvergemisches, die in 25 ml Wasser suspendiert wurde, peroral behandelt.

**Beispiel 2**

Es wurden 55 g 2,4-Diamino -5- [3', 4', 5'-tri-(methoxy)-benzyl]-pyrimidin {Trimethoprim}<a)>, 0,5 g 2-Chlor -10- [3-(dimethylamino)-propyl]-phenothiazin {Chlorpromazinhydrochlorid} <b)>, 15 g 2-(Methyl)-5,7-di-(chlor) -8- (hydroxy)-chinolin {Chlorchinaldol} <c)>, 40 g Wismutsubsalicylat, 50 g Glucose, 20 g Nicotinamid, 45,5 g Aluminiumsilikat, 10 g Hexamethylentetramin und 4 g Siliciumdioxid in einer Kugelmühle vermahlen. Das erhaltene Pulvergemisch wurde vor der Verabreichung in der im Beispiel 1 beschriebenen Weise in Kamillentee oder Wasser suspendiert.

**Patentansprüche für die Vertragsstaaten BE, CH, DE, FR, GB, IT, LI, NL, SE**

1. Arzneimittel und/oder Futtermittelkonzentrate mit antibakterieller Wirkung, mit einem Gehalt an

a) 2,4-Diamino-5-[3',4',5'-tri-(methoxy)-benzyl]-pyrimidin {Trimethoprim},

gegebenenfalls zusammen mit 1 oder mehr in Arzneimitteln und/oder Futtermittelkonzentraten üblichen Konfektionierungsmittel(n) und/oder 1 oder mehr Salicylsäurederivat(en), dadurch gekennzeichnet, dass sie als weitere Wirkstoffe

b) 2-Chlor -10 [3-(dimethylamino)-propyl]-phenothiazin {Chlorpromazin} und/oder 1 oder mehr physiologisch verträgliche[s] Säureadditionssalz(e) desselben und

c) 2-(Methyl)-5,7-di-(chlor) -8- (hydroxy)-chinolin {Chlorchinaldol} enthalten.

2. Arzneimittel und/oder Futtermittelkonzentrate nach Anspruch 1, dadurch gekennzeichnet, dass sie als physiologisch verträgliches Säureadditionssalz von 2-Chlor -10- [3-(dimethylamino)-propyl]-phenthiazin {Chlorpromazin}

<b)> 2-Chlor -10- [3-(dimethylamino)-propyl]-phenothiazin-hydrochlorid {Chlorpromazinhydrochlorid} enthalten.

3. Arzneimittel und/oder Futtermittelkonzentrate nach Anspruch 1 oder 2, dadurch gekennzeichnet, dass das Gewichtsverhältnis von a):b):c) 6:1:6 bis 110: 1:100 ist.

4. Verfahren zur Herstellung der Arzneimittel und Futtermittelkonzentrate nach Anspruch 1 bis 3, dadurch gekennzeichnet, dass man die Wirkstoffe und gegebenenfalls das beziehungsweise die Konfektionierungsmittel miteinander vermischt.

5. Erzeugnisse, enthaltend 2,4-Diamino -5- [3', 4', 5'-tri-(methoxy)-benzyl]-pyrimidin {Trimethoprim} <a)> und gegebenenfalls

1 oder mehr in Arzneimitteln und/oder Futtermittelkonzentraten übliche Konfektionierungsmittel, dadurch gekennzeichnet, dass sie ausser a) als weitere Wirkstoffe

2-Chlor -10- [3-(dimethylamino)-propyl]-phenothiazin {Chlorpromazin} <b)> und/oder 1 oder mehr physiologisch verträgliche[s] Säureadditionssalz(e) desselben <b)>

2-(Methyl)-5,7-di-(chlor -8- (hydroxy)-chinolin {Chlorchinaldol} <c)>

als Kombinationspräparat zur gleichzeitigen, getrennten oder zeitlich abgestuften Anwendung in der antibakteriellen Therapie enthalten.

6. Erzeugnisse nach Anspruch 5, dadurch gekennzeichnet, dass sie als physiologisch verträgliches Säureadditionssalz von 2-Chlor -10- [3-(dimethylamino)-propyl]-phenothiazin {Chlorpromazin} <b)> 2-Chlor -10- [3-(dimethylamino)-propyl]-phenothiazin-hydrochlorid {Chlorpromazinhydrochlorid} enthalten.

7. Erzeugnisse nach Anspruch 5 oder 6, dadurch gekennzeichnet, dass das Gewichtsverhältnis von a):b):c) 6:1:6 bis 110:1:100 ist.

**Patentansprüche für den Vertragsstaat: AT**

1. Verfahren zur Herstellung von Arzneimitteln und/oder Futtermittelkonzentraten, mit antibakterieller Wirkung mit einem Gehalt an

a) 2,4-Diamino -5- [3', 4', 5'-tri-(methoxy)-benzyl]-pyrimidin {Trimethoprim},

gegebenenfalls zusammen mit 1 oder mehr in Arzneimitteln und/oder Futtermittelkonzentraten üblichen Konfektionierungsmittel(n) und/oder 1 oder mehr Salicylsäurederivat(en), dadurch gekennzeichnet, dass ihnen als weitere Wirkstoffe

b) 2-Chlor -10- [3-(dimethylamino)-propyl]-phenothiazin {Chlorpromazin} und/oder 1 oder mehr physiologisch verträgliche(s) Säureadditionssalz(e) desselben und

c) 2-(Methyl)-5,7-di-(chlor) -8- (hydroxy)-chinolin {Chlorchinaldol} zugemischt werden.

2. Verfahren nach Anspruch 1, dadurch gekennzeichnet, dass als physiologisch verträgliche Säureadditionssalze von 2-Chlor -10- [3-(dimethylamino)-propyl]-phenthiazin {Chlorpromazin} <b)> 2-Chlor -10- [3-(dimethylamino)-propyl]-phenothiazinhydrochlorid {Chlorpromazinhydrochlorid} verwendet wird.

3. Verfahren nach Anspruch 1 oder 2, dadurch gekennzeichnet, dass ein Gewichtsverhältnis von a):b):c) von 6:1:6 bis 110:1:100 eingehalten wird.

### Revendications pour les Etats Contractants BE, CH, DE, FR, GB, IT, LI, NL, SE

1. Médicaments et/ou concentrés de fourrage à effet antibactérien, renfermant:

a) de la 2,4-diamino -5- [3',4',5'-tri-(méthoxy)-benzyl]-pyrimidine (triméthoprime), éventuellement en même temps qu'un ou plusieurs produit(s) de confection usuel(s) dans les médicaments et/ou les concentrés de fourrage et/ou un ou plusieurs dérivé(s) de l'acide salicylique, caractérisés en ce qu'ils renferment, comme autres substances actives:

b) de la 2-chloro -10- [3-(diméthylamino)-propyl]-phénothiazine (chloropromazine) et/ou un ou plusieurs sel(s) d'addition d'acides physiologiquement compatible(s) de celle-ci, et

c) de la 2-méthyl-5,7-di-(chloro) -8- (hydroxy)-quinoléine (chloroquinaldol).

2. Médicaments et/ou concentrés de fourrage selon la revendication 1, caractérisés en ce qu'ils renferment, comme sel d'addition d'acides physiologiquement compatible de la 2-chloro -10- [3-(diméthylamino)-propyl]-phénothiazine (chloropromazine) <b)>, du chlorhydrate de 2-chloro -10- [3-(diméthylamino)-propyl]-phénothiazine (chlorhydrate de chloropromazine).

3. Médicaments et/ou concentrés de fourrage selon la revendication 1 ou la revendication 2, caractérisés en ce que la proportion pondérale de a):b):c) est de 6: 1:6 à 110:1:100.

4. Procédé de préparation des médicaments et concentrés de fourrage selon les revendications 1 à 3, caractérisé en ce qu'on mélange ensemble les substances actives et éventuellement le ou les produits de confection.

5. Produits renfermant:

de la 2,4-diamino -5- [3', 4', 5'-tri-(méthoxy)-benzyl]-pyrimidine (triméthoprime) <a)>, et éventuellement un ou plusieurs produit(s) de confection usuel(s) dans les médicaments et/ou les concentrés de fourrage, caractérisés en ce qu'ils renferment, en dehors de a), comme autres substances actives,

de la 2-chloro -10- [3-(diméthylamino)-propyl]-phénothiazine (chloropromazine) <b)> et/ou un ou plusieurs sel(s) d'addition d'acides physiologiquement compatible(s) de celle-ci <b)>, et

de la 2-(méthyl)-5,7-di-(chloro) -8- (hydroxy)-quinoléine (chloroquinaldol) <c)>, sous la forme de produit combiné pour l'application simultanée, séparée ou échelonnée dans le temps en thérapie antibactérienne.

6. Produits selon la revendication 5, caractérisés en ce qu'ils renferment, comme sel d'addition d'acides physiologiquement compatible de la 2-chloro -10- [3-(diméthylamino)-propyl]-phénothiazine (chloropromazine <b)>, du chlorhydrate de 2-chloro -10- [3-(diméthylamino)-propyl]-phénothiazine (chlorhydrate de chloropromazine).

7. Produits selon la revendication 5 ou la revendication 6, caractérisés en ce que la proportion pondérale de a):b):c) est de 6:1:6 à 110:1:100.

### Revendications pour l'Etat contractant AT

1. Procédé de préparation des médicaments et concentrés de fourrage à effet antibactérien, renfermant:

a) de la 2,4-diamino -5- [3',4',5'-tri-(méthoxy)-benzyl]-pyrimidine(triméthoprime),

éventuellement en même temps qu'un ou plusieurs produit(s) de confection usuel(s) dans les médicaments et/ou les concentrés de fourrage et/ou un ou plusieurs dérivé(s) de l'acide salicylique, caractérisés en ce qu'on mélange comme autres substances actives:

b) de la 2-chloro -10- [3-(diméthylamino)-propyl]-phénothiazine (chloropromazine) et/ou un ou plusieurs sel(s) d'addition d'acides physiologiquement compatible(s) de celle-ci, et

c) de la 2-méthyl-5,7-di(chloro) -8- (hydroxy)-quinoléine (chloroquinaldol).

2. Procédé selon la revendication 1, caractérisés en ce que comme sel d'addition d'acides physiologiquement compatible de la 2-chloro -10- [3-(diméthylamino)-propyl]-phénothiazine (chloropromazine) <b)> le chlorhydrate · de 2-chloro -10- [3-(diméthylamino)-propyl]-phénothiazine (chlorhydrate de chloropromazine) est utilisé.

3. Procédé selon la revendication 1 ou 2, caractérisés en ce qu'une proportion pondérale de a):b):c) de 6:1:6 à 110:1:100 est utilisé.

### Claims for the Contracting States BE, CH, DE, FR, GB, IT, LI, NL, SE

1. Pharmaceuticals and/or feed stuff concentrates having an antibacterial effect containing

a) 2,4-diamino -5- [3',4',5'-tri-(methoxy)-benzyl]-pyrimidine {trimethoprime},

optionally together with 1 or more auxiliary agent(s) being conventionally used in pharmaceuticals and/or feed stuff concentrates, and/or 1 or more salicylic acid derivative(s), characterized in that as additional active ingredients they contain

b) 2-chloro -10- [3-(dimethylamino)-propyl]-phenothiazine {chloropromazine} and/or 1 or more physiologically acceptable acid addition salt(s) thereof, and

c) 2-(methyl)-5,7-di-(chloro) -8- (hydroxy)-chinoline {chlorochinaldol}.

2. Pharmaceuticals and/or feed stuff concentrates according to claim 1, characterized in that as physiologically acceptable acid addition salt of 2-chloro -10- [3-(dimethylamino)-propyl]-phenothiazine {chloropromazine} <b)> they contain 2-chloro -10- [3-(dimethylamino)-propyl]-phenothiazine-hydrochloride {chloropromazine hydrochloride}.

3. Pharmaceuticals and/or feed stuff concentrates according to claim 1 or 2, characterized in that the weight ratio a):b):c) is 6:1:6 to 110:1:100.

4. A process for preparing the pharmaceuticals and feed stuff concentrates according to claims 1 to 3, characterized in that the active ingredients and optionally the auxiliary agent(s) are mixed.

5. Products, containing

2,4-diamino -5- [3', 4', 5'-tri-(methoxy)-benzyl]-pyrimidine {trimethoprime} <a)>, and optionally

1 or more auxiliary agent(s) being conventionally used in pharmaceuticals and/or feed stuff concentrates,

characterized in that besides of a) they contain as additional active ingredients 2-chloro -10- [3-(dimethylamino)-propyl]-phenothiazine {chloropromazine} <b)> and/or 1 or more physiologically acceptable acid addition salt(s) thereof <b)> and 2-(methyl)-5,7-di-(chloro) -8- (hydroxy)-chinoline {chlorochinaldol} <c)>

as combination preparation for the simultaneous, separate or timely graduated use in the antibacterial therapy.

6. Products according to claim 5, characterized in that as physiologically acceptable acid addition salt of 2-chloro -10- [3-(dimethylamino)-propyl]-phenothiazine {chloropromazine} <b)> they contain 2-chloro -10- [3-(dimethylamino)- propyl]-phenothiazine-hydrochloride {chloro- promazine hydrochloride}.

7. Products according to claim 5 or 6, characterized in that the weight ratio a):b):c) is 6:1:6 to 110:1:100.

**Claims for the Contracting State AT**

1. A process for preparing pharmaceuticals and/or feed stuff concentrates with antibacterial effect containing

a) 2,4-diamino -5- [3',4',5'-tri-(methoxy)-benzyl]-pyrimidine {trimethoprime},

optionally together with 1 or more auxiliary agent(s) being conventionally used in pharmaceuticals and/or feed stuff concentrates, and/or 1 or more salicylic acid derivative(s), characterized in that as additional active ingredients

b) 2-chloro -10- [3-(dimethylamino)-propyl]- phenothiazine {chloropromazine} and/or 1 or more physiologically acceptable acid addition salt(s) thereof and

c) 2-methyl-5,7-di-(chloro) -8- (hydroxy)- chinoline {chlorochinaldol} are added thereto.

2. A process according to claim 1, characterized in that as physiologically acceptable acid addition salt of 2-chloro -10- [3-(dimethylamino)-propyl]-phenothiazine {chloropromazine} <b)> 2-chloro -10- [3-(dimethylamino)-propyl]-phe- nothiazinhydrochloride {chloropromazine hydrochloride} is used.

3. A process according to claim 1 or 2, characterized in that a weight ratio a):b):c) of 6:1:6 to 110:1:100 is maintained.